# EUROPEAN PATENT SPECIFICATION

(11) **EP 0 836 475 B1**
(45) Date of publication and mention of the grant of the patent: **07.11.2001**
(21) Application number: 96921053.3
(22) Date of filing: 01.07.1996
(51) Int. Cl.: A61K 9/14, A61K 9/20

(54) **CONTROLLED RELEASE FORMULATIONS FOR POORLY SOLUBLE DRUGS**
FORMULIERUNGEN MIT GESTEUERTER FREISETZUNG FÜR SCHWERLÖSLICHE ARZNEISTOFFE
FORMULATIONS A LIBERATION LENTE POUR MEDICAMENTS FAIBLEMENT SOLUBLES

(30) Priority: 03.07.1995 IE 950492; 06.07.1995 US 897 P
(43) Date of publication of application: 22.04.1998
(73) Proprietor: ELAN CORPORATION, Plc, Athlone County Westmeath (IE)
(72) Inventor: CLANCY, Maurice, Joseph, Anthony, Athlone, County Westmeath (IE); CUMMING, Kenneth, Iain, Dublin 1 (IE); MYERS, Michael, Ashburn, VA 22011 (US)
(74) Representative: Ryan, Anne Mary
(86) International application number: IE9600039
(87) International publication number: WO9702017

(56) References cited:
- EP-A- 0 317 780
- DRUG DEVELOPMENT AND INDUSTRIAL PHARMACY, vol. 18, no. 16, 1992, pages 1719-1739, XP000612180 HAWLEY, A. R. ET AL.: "Physical and chemical characterization of thermosoftened bases for molten filled hard gelatin capsule formulations"

## Description

### Technical Field

This invention relates to controlled release formulations for poorly soluble drugs for oral administration.

### Background Art

Various controlled absorption/release pharmaceutical formulations are available which have a particular dissolution pattern, resulting in a controlled absorption of the active substance and, therefore, more effective medication. The use of many active substances in therapy is complicated by solubility problems. In the case of some insoluble drugs various methods have been used to enhance solubility such as micronisation, formation of amorphous co-precipitates, or preparation of inclusion complexes using materials such as cyclodextrins. Various surfactants have also been utilised to enhance the solubility of various insoluble compounds using different formulation strategies.

Some drugs, such as nifedipine, are non-ionisible and show a low solubility throughout all regions of the gastrointestinal tract. Other drugs are either basic or acidic in character and show pH dependent limited solubility in different regions of the gastrointestinal tract. One such example is cisapride, which is basic in nature and has only relatively low solubility in acid conditions in the upper part of the gastrointestinal tract and very poor solubility as it passes further down the gastrointestinal tract.

Our EP 0 232 155 B1 and EP 0 274 176 describe adsorbates for use in drug delivery systems. EP 0 232 155 B1 describes adsorbates consisting of a mixture of a pharmaceutically useful active ingredient and an inactive substance adsorbed on a cross-linked polymer which is granulated and then blended with a polymer or mixture of polymers to form a sustained release drug delivery matrix system. EP 0 274 176 describes sustained release capsule and tablet formulations based on an adsorbate of a mixture of a pharmaceutically useful dihydropyridine and a polyvinylpyrrolidone having an average molecular weight greater than 55,000 adsorbed on a cross-linked polyvinylpyrrolidone, the adsorbate being blended with at least one polymer which gels in the presence of water so as to obtain a sustained release effect.

EP-A-0 317 780 describes 1,4-dihydropyridine complexes with polyoxypropylene-polyoxyethylene complexes and their use in forming sustained and quick release dosage formulations. The formulations are formed by combining the complexes with one or more water soluble cellulose derivatives with molecular weights which provide a viscosity in water of 9 to 30 cps and 3 to 8 cps, respectively at 20°C as a 2%w/w aqueous solution. In vitro release data is given for the different types of formulations. When hydroxypropylmethylcellulose (HPMC) having a viscosity of 15 cps was used as the cellulose derivative an in vitro release of nifedipine of ~ 25% was observed at 6 hours. The in vitro release at six hours was greater than 80% for HPMC with a viscosity of 6 cps or a 50:50 mixture of HPMC with viscosities of 6 cps and 15 cps.

Drug Development and Industrial Pharmacy (1992) 18,16, 1719-1739 describes the investigation of potential bases including the polyoxypropylene-polyoxyethylene copolymer Lutrol-F68 for the preparation of fusion formed solid dispersions for molten filling into hard gelatin capsules. The investigations included dissolution studies on various drug/base formulations. When the in vitro release profile for ibuprofen from Lutrol-F68 was investigated a T50 value of approximately 30 min. was observed.

It is an object of the present invention to provide an improved drug delivery system wherein the bioavailability of an otherwise poorly bioavailable active ingredient is enhanced and effectively controlled.

It is a further object of the present invention to provide controlled release dosage forms of poorly soluble active ingredient which provide therapeutic levels for a period of up to 24 hours or longer, dependent on the half-life of the active ingredient.

### Disclosure of Invention

The invention provides a controlled release formulation for oral administration, comprising a solid dispersion of an active ingredient, which has poor aqueous solubility, in a hydrophilic poloxamer polymer, said solid dispersion being blended with a water swellable hydroxypropylmethylcellulose, a 2% solution of which has a viscosity in the range 100-100,000 centipoise, to form a hydrogel matrix, and one or more tabletting ingredients to form a tablet core, said tablet core as such or following coating of the core with a polymeric coating being effective to achieve therapeutic levels of said active ingredient over extended periods of time following oral administration.

It is found that incorporation of a poorly soluble active ingredient into a solid dispersion in a formulation according to the invention achieves a significant level of solubility/wettability enhancement, as well as achieving therapeutic levels of said active ingredient over extended periods *in vivo.*

To accommodate the varying solubility of different active ingredients of the type referred to above, the solid dispersion may need to include one or more ingredients to further improve the solubility/wettability enhancement of the active ingredient.

Accordingly, the solid dispersion may include a surfactant component. The surfactant may be an anionic, cationic or non-ionic surfactant. Preferred surfactant components are selected from sodium lauryl sulphate, a sodium carboxylate, an alkyl sulphate, a polyethylene glycol ester, a polyethylene ether, a sorbitan ester, an ethoxylated sorbitan ester and an alkyl trimethylammonium halide and a mixture thereof.

Alternatively, the solid dispersion may include an acid component. Preferred acid components are selected from adipic acid, ascorbic acid, citric acid, fumaric acid, malic acid, succinic acid and tartaric acid.

Further, alternatively, the solid dispersion may include a base component. Preferred base components are selected from calcium carbonate, calcium hydroxide, magnesium hydroxide, sodium bicarbonate, sodium carbonate, sodium citrate and sodium hydroxide.

Preferably, the surfactant, acid or base component is present in a ratio of 0.01:1.0 to 5.0:1.0 by weight of the active ingredient.

Any such surfactant, acid or base components are hereinafter collectively referred to as auxiliary agents.

Further, preferably, the active ingredient and the poloxamer are present in an amount of 0.1:1.0 to 10.0:1.0 by weight.

By poloxamer herein is meant also a combination of two or more poloxamers.

The poloxamer polyols are a series of closely related block copolymers of ethylene oxide and propylene oxide.

More specifically, the poloxamer polyols are α-hydro-ω-hydroxypoly(oxyethylene)poly(oxypropylene)poly(oxyethylene) block copolymers, more generally known as polyethylene - propylene glycol copolymer or polyoxyethylene - polyoxypropylene copolymer.

Preferred poloxamers are those which contain between 60% and 90%, more especially between 70% and 80%, by weight of the polyoxyethylene portion.

The polyoxyethylene segment is hydrophilic whilst the polyoxypropylene segment is hydrophobic. All of the poloxamers are chemically similar in composition, differing only in the relative amounts of propylene oxide and ethylene oxide added during manufacture. The hydrophilic segment can comprise between 15 and 90% of the molecule. As indicated above, those recommended for use in accordance with the invention have between 60% and 90%, more especially between 70% and 80%, by weight of the hydrophilic polyoxyethylene segment or sequence. Such poloxamer polyols, hereinafter referred to as poloxamers, are known by the trade names Lutrol, Monolan and Pluronic.

Poloxamers are also defined by a number. The first two digits of the number, when multiplied by 100, correspond to the approximate average molecular weight of the polyoxypropylene (POP) portion of the molecule. The third digit, when multiplied by 10, corresponds to the percentage by weight of the polyoxyethylene (POE) portion. When called by their Pluronic name, the first two digits, when multiplied by 1000 indicate the total molecular weight, and the third digit, multiplied by 10, represents the approximate percentage of POE in the molecule. The associated capital letter indicates the physical state: L = liquid, P = paste, and F = solid. For further information, reference can be made to Pharmaceutical Technology Europe May 1994.

The preferred poloxamers for use in accordance with the invention are the F series. Especially preferred poloxamers of the F series are F68, F108 and F127, especially those sold under the trade marks Lutrol F 68, Pluronic F 108 and Lutrol F 127 by BASF.

Further information on these poloxamers can be obtained from the technical information sheets supplied by BASF (Ireland) Limited.

The poloxamer is melted and then the active ingredient and any auxiliary agents(s) are dispersed in the molten poloxamer.

The poloxamer is suitably melted in a stainless steel container. To the molten mass the active ingredient, and any auxiliary agent(s) and the inert filler, if used, are added slowly over time. The mixture is stirred while it is cooled and milled to a median particle size in the range 30-300 µm.

Alternatively, the active ingredient, any auxiliary agent(s), and the poloxamer are dissolved in an organic solvent or solvents; the solvent is evaporated and the molten poloxamer is cooled and milled to a median particle size in the range 30-300 µm.

It has been found that various poorly soluble active ingredients are readily dispersible in the melt forms of the poloxamers used in accordance with the invention. When cooled the mixture of active ingredient, auxiliary agent(s), if present, and poloxamer forms a dry, hard solid which can be easily ground or milled. It is for this reason that the poloxamers were chosen herein as the polymer base for the solid dispersion.

The solid dispersion according to the invention can include excipient agents, such as an inert filler. The inert filler is suitably a water soluble inert filler. An example of an inert filler is lactose, more especially lactose monohydrate. The inclusion of an inert filler such as lactose may have the effect of lowering the melting point of the poloxamer.

The incorporation of an inert filler is found to have no detrimental impact on the solubility obtained. However, it does significantly improve overall processability and lead to a fine, powder material which is very suitable for compression into tablets. The inert filler is suitably used in an amount of 3-35% by weight.

The active ingredient can be any poorly soluble drug of the type mentioned above. Examples of such drugs are cisapride, cyclosporin, diclofenac, felodipine, ibuprofen, indomethacin, nicardipine, nifedipine, terfenadine and theophylline.

An aim of the present invention is to provide poorly soluble active ingredients such as cisapride in a readily solublised and absorbable form at distal sites in the gastrointestinal tract so as to achieve continuing and extended absorption. The aqueous solubility of cisapride, which is inherently low and significantly pH dependent, becomes limiting as cisapride is normally presented to more distal sites in the gastrointestinal tract where the water content and the pH adversely impact on the solubility of cisapride. Although not wishing to be bound by any theoretical explanation of the invention, it is postulated that the increased solubility of cisapride obtained through the use of a solid dispersion as hereinabove described results in an optimal micro-environment which promotes the availability of solublised and absorbable cisapride. Furthermore, the presence of a hydroxy acid in the solid dispersion, combined with the controlled release system employed, results in a gradual availability of the hydroxy acid to promote the solubilisation of the cisapride over the bulk of the release rate curve.

Similar results are obtained with other poorly soluble active ingredients as hereinafter described.

The solid oral dosage form according to the invention can be in the form of capsules.

Thus, capsules in accordance with the invention can contain the formulation according to the invention in a multi-particulate form such as mini-tablets.

Suitably, the capsules will include an amount of mini-tablets for immediate release of active ingredient comprising a solid dispersion as hereinbefore defined.

Various multi-particulate forms or a small number of discrete units can be presented in capsule form.

The capsules can be hard or soft gelatin capsules.

The tablets consist of a tablet core defined by a solid dispersion as hereinbefore described dispersed in a hydrogel matrix. In this form of tablet, the solid dispersion is compressed into a dosage form containing a polymer or mixture of polymers which when wet will swell to form a hydrogel. The rate of release of the active ingredient from this dosage form is controlled both by diffusion from the swollen tablet mass and by erosion of the tablet surface over time. The rate of release of the active ingredient may be controlled both by the amount of polymer *per* tablet and by the inherent viscosities of the polymers used.

The water swellable hydroxypropylmethylcellulose (HPMC) polymer is suitably present in an amount of 5-75% by weight. By hydroxypropylmethylcellulose herein is meant a combination of hydroxypropylmethylcelluloses.

As indicated above suitably the HPMC polymer will be present in an amount of from 5% to 75% by weight, more especially from 10% to 60% by weight.

An especially preferred type of HPMC for use in accordance with the invention is HPMC sold under the trade mark Methocel.

The amount of HPMC polymer used will be dependent on the viscosity thereof. In general, the higher the viscosity of the polymer, the lesser amount of the polymer required to give the desired release properties.

Suitable Methocels are Methocel K15M, a 2% solution of which has a viscosity of 15,000 centipoise. Other suitable Methocels include Methocel K4M, K100M, K100LV or E, F and J grades, depending on the release characteristics desired.

As indicated above, the use of hydrogel matrices causes the tablet to swell and release drug in a controlled manner by erosion of the tablet surface and diffusion from the tablet mass. Cellulose polymers with different inherent viscosities are preferably used to control the dissolution rate.

HPMC hydrogels are linear macromolecules which swell in water or biological fluids. Drug release in such systems can be basically modified by varying the following parameters:
Type and viscosity grade of polymers; and
Actual concentrations.

The polymer type chosen in accordance with the invention is determined primarily by the solubility characteristics of the solid dispersion to be compressed. The viscosity grade is dictated by the desired rate of release of active ingredient from the tablet mass.

Tablet hardness is also a parameter to be considered in the case of hydrogel tablets. Suitably the mean hardness of the tablets is in the range 60-260 N.

For tabletting purposes, it will also be usual to use a diluent or compacting agent such as microcrystalline cellulose, more especially microcrystalline cellulose sold under the trade mark Avicel, for example, Avicel pH101.

Other excipient agents may include lubricants such as magnesium stearate and a glidant such as colloidal silicon dioxide sold under the trade mark Aerosil.

The solid oral dosage form according to the invention can also be in the form of tablets comprising a core containing a solid dispersion as hereinbefore described surrounded by a multi-porous, rate-controlling membrane. Suitably the solid dispersion is in the form of an instant release tablet core which is adapted for direct compression followed by coating with the rate-controlling membrane. A primary consideration with such formulations is the selection of suitable tablet ingredients to impart the desired effect while compressing easily. In order to achieve an instant release tablet core the solid dispersion is suitably blended with standard tablet excipients such as a standard compressing base, a compressible sugar, a solubilising agent and a lubricating agent. In this type of dosage form, release of the active ingredient is controlled *via* a diffusion mechanism.

Tablet hardness and friability play an important role in the characterisation of the membrane coated tablet. It is essential that the core tablets are sufficiently rugged to withstand the coating process.

In order to obtain the desired release profile suitable for once-daily administration, the rate-controlling membrane will suitably contain a major proportion of a pharmaceutically acceptable film-forming, water insoluble polymer and optionally a minor proportion of a pharmaceutically acceptable film-forming, water soluble polymer.

The term water soluble polymer as used herein includes polymers which are freely permeable to water, whilst the term water insoluble polymer as used herein includes polymers which are slightly permeable to water, as hereinafter indicated.

Preferably the water soluble polymer in the membrane, if present, is selected from polyvinyl alcohol, polyvinylpyrrolidone, methyl cellulose, hydroxypropyl cellulose, hydroxypropylmethyl cellulose, agar, carrageenan, xanthan or polyethylene glycol or a mixture thereof.

The incorporation of various hydrophilic agents into the polymer coating so as to form channels in said coating can be used and in general leads to a more linear release rate. Such hydrophilic agents include fumaric acid, citric acid, tartaric acid, sodium citrate, sodium bicarbonate, sodium fumarate, sodium carbonate, monosaccharides and disaccharides. An especially suitable monosaccharide is glucose.

Alternatively, the water soluble polymer in the membrane can be replaced by a polymeric material which is freely permeable to the active ingredient and water and comprises a copolymer of acrylic and methacrylic acid esters.

A suitable polymer which is freely permeable to various poorly soluble active ingredients and water is a polymer sold under the Trade Mark EUDRAGIT RL.

Preferably, the water insoluble polymer in membrane is selected from ethylcellulose, cellulose acetate, cellulose propionate (lower, medium or higher molecular weight), cellulose acetate propionate, cellulose acetate butyrate, cellulose acetate phthalate, cellulose triacetate, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), poly(ethylene), poly(ethylene) low density, poly(ethylene) high density, poly(propylene), poly(ethylene oxide), poly(ethylene terephthalate), poly(vinyl isobutyl ether), poly(vinyl acetate), poly(vinyl chloride) or polyurethane or a mixture thereof.

The water insoluble polymer of the membrane may also comprise naturally occurring polymers or resins.

Thus other preferred water insoluble polymers are selected from a naturally occurring polymer selected from shellac, chitosan, gum juniper or a mixture thereof.

Alternatively, the water insoluble polymer in the membrane can be replaced by a polymeric material which is slightly permeable to the active ingredient and water and comprises a copolymer of acrylic and methacrylic acid esters.

A suitable polymer which is slightly permeable to various poorly soluble active ingredients and water is a polymer sold under the Trade Mark EUDRAGIT RS or a polymer whose permeability is pH dependent and sold under the Trade Mark EUDRAGIT L, EUDRAGIT S or EUDRAGIT E. Especially preferred polymers in this category are EUDRAGIT S.

EUDRAGIT polymers are polymeric lacquer substances based on acrylates and/or methacrylates. The polymeric materials sold under the Trade Mark EUDRAGIT RL and EUDRAGIT RS are acrylic resins comprising copolymers of acrylic and methacrylic acid esters with a low content of quaternary ammonium groups and are described in the "EUDRAGIT" brochure of Messrs. Rohm Pharma GmbH (1984) wherein detailed physical-chemical data of these products is given. The ammonium groups are present as salts and give rise to the permeability of the lacquer films. EUDRAGIT RL and RS are freely permeable (RL) or slightly permeable (RS), respectively, independent of pH.

EUDRAGIT S is an anionic polymer synthesized from methacrylic acid and methacrylic acid methyl ester. It is insoluble in acids and pure water. It becomes soluble in a neutral to weakly alkaline milieu by forming salts with alkalis. The permeability of EUDRAGIT S is pH dependent. Above pH 6.0 the polymer becomes increasingly permeable. EUDRAGIT S is described in the "EUDRAGIT S" brochure of Messrs. Rohm Pharma GmbH (1986) wherein detailed physical-chemical data of the product is given.

The coating solution/suspension of the polymeric material comprises one or more polymer(s) dissolved/suspended in a suitable solvent or mixture of solvents. The concentration of the polymeric material in the coating solution/suspension is determined by the viscosity of the final solution/suspension. The addition of a plasticizing agent to the polymeric solution/ suspension may be necessary depending on the formulation to improve the elasticity and also the stability of the polymer film and to prevent changes in the polymer permeability over prolonged storage.

Such changes could affect the drug release rate. Suitable plasticizing agents include polyethylene glycol, propylene glycol, glycerol, triacetin, dimethyl phthalate, diethyl phthalate, dibutyl phthalate, dibutyl sebacate, triethyl citrate, tributyl citrate, triethyl acetyl citrate, castor oil and varying percentages of acetylated monoglycerides.

Coating the active tablet core with a differentially permeable membrane allows active dissolution and diffusion from the micro-environment of the active core. Especially, suitable polymers are cellulose and poly(methacrylate) based polymers for use as coating agents. Polymer coating is most suitably performed in a C.F. 360 granulator.

Preferred solvents for use in polymer application/coating include acetone, isopropyl alcohol and industrial methylated spirit.

Based on the information given above other ways of incorporating the solid dispersion hereinabove described to achieve improved solubilisation/wettability and improved controlled absorption/release will be apparent to one skilled in the art.

### Brief Description of the Drawings

Fig. 1 is a plot of plasma levels of nifedipine (ng/ml) *versus* time (hours) after administration for the formulations of Examples 1 and 2;
Fig. 2 is a plot of plasma levels of nifedipine (ng/ml) *versus* time (hours) after administration for the formulations of Examples 3 and 4;
Fig. 3 is a plot of plasma levels of nifedipine (ng/ml) *versus* time (hours) after administration for the formulations of Example 5 and the reference product;
Fig. 4 is a plot of plasma levels of cisapride (ng/ml) *versus* time (hours) after administration for the tablet formulations of Examples 6 and 7 in which: curve a) corresponds to the tablet formulation of Example 6; and curve b) corresponds to the tablet formulation of Example 7; and
Fig. 5 is a plot of plasma levels of cisapride (ng/ml) *versus* time (hours) after administration for the formulations of Examples 8 -11 and the reference product.

### Modes for Carrying Out the Invention

This invention will be further illustrated by the following Examples.

### Example 1 (Comparative Example)

### Preparation of tablet

A tablet formulation was prepared by blending the following tablet ingredients in the indicated proportions:

| Ingredient | Weight % |
|---|---|
| Nifedipine | 12.82 |
| Methocel K15M | 64.10 |
| Avicel PH101 | 22.22 |
| Magnesium stearate | 0.86 |

The tablet blend was tabletted to a mean hardness of 55N in a Killian RTS tablet press. The *in vitro* dissolution for the tablets was determined in a USP Apparatus II (paddles) at 100 r.p.m. in 1.25% SLS aqueous solution at pH 6.8 medium, at a volume of 900ml and a temperature of 37°C ± 0.5.

The following results were obtained:

| Time(hours) | % Release |
|---|---|
| 1.0 | 2.5 |
| 2.0 | 8.7 |
| 4.0 | 21.7 |
| 6.0 | 30.3 |
| 8.0 | 43.3 |
| 10.0 | 52.5 |
| 24.0 | 101.0 |

### Example 2

### Preparation of solid dispersion

Lutrol F127 (500g) was melted by heating to 80°C. Nifedipine (500g) was gradually added to, and dispersed in, the molten Lutrol. Mixing was continued for 2hr before the solid dispersion was allowed to cool at room temperature, followed by milling.

### Preparation of tablet

A tablet formulation was prepared by blending the solid dispersion with the following tablet ingredients in the indicated proportions:

| Ingredient | Weight % |
|---|---|
| Solid dispersion | 23.43 |
| Methocel K15M | 56.30 |
| Avicel PH101 | 19.52 |
| Magnesium stearate | 0.75 |

The tablet blend was tabletted to a mean hardness of 52N in a Killian RTS tablet press. The *in vitro* dissolution for the tablets was determined under the conditions as specified in Example 1. The following results were obtained:

| Time(hours) | % Release |
|---|---|
| 1.0 5.3 | |
| 2.0 8.2 | |
| 4.0 20.3 | |
| 6.0 31.6 | |
| 8.0 42.3 | |
| 10.0 | 53.6 |
| 24.0 | 110.8 |

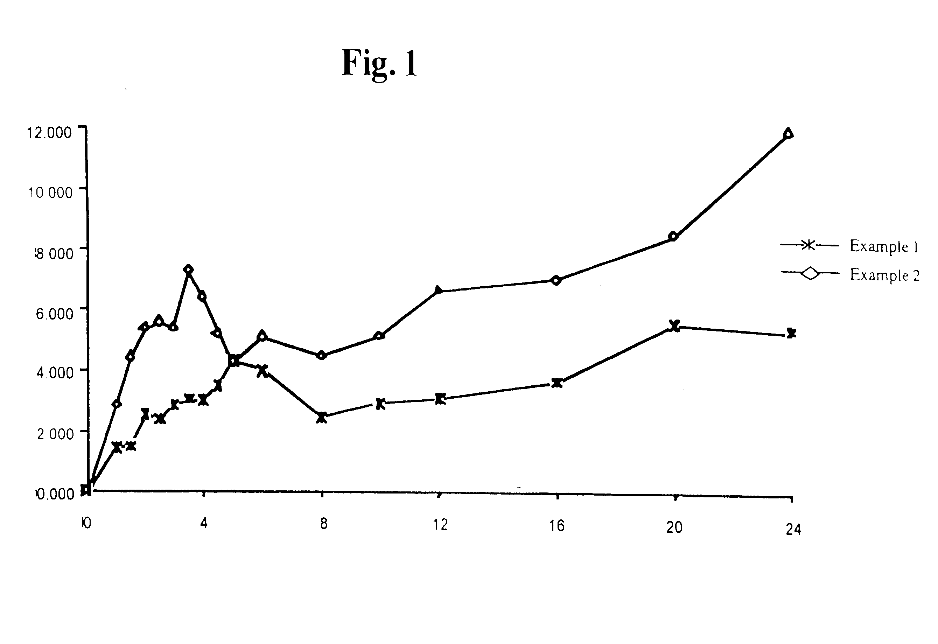

A comparison of the pharmacokinetic data for the formulations prepared in Examples 1 and 2 is shown in Table 1 and accompanying Fig. 1.

**Table 1**

| Parameter | Example 1 | Example 2 |
|---|---|---|
| AUC*₍₀₋₂₄₎ | 85.54 ± 54.12 | 153.10 ± 75.85 |
| Cmax** | 8.58 ± 4.32 | 16.10 ± 9.21 |
| Tmax*** | 12.89 ± 9.05 | 12.78 ± 9.25 |

| | | |
|---|---|---|
| * Area under the plasma drug level *versus* time curve | | |
| ** Maximum plasma concentration of drug attained | | |
| *** The time at which C max is attained | | |

The effect of including the solid dispersion (Example 2) with the attendant increase in bioavailability is clear from a comparison of Example 1 (raw material alone) and Example 2.

### Example 3

### Preparation of solid dispersion

Lutrol F127 (1500g) was melted by heating to 80°C. Nifedipine (500g) was gradually added to, and dispersed in, the molten Lutrol. Mixing was continued for 2hr before the solid dispersion was allowed to cool at room temperature, followed by milling.

### Preparation of tablet

A tablet formulation was prepared by blending the solid dispersion with the following tablet ingredients in the indicated proportions:

| Ingredient | Weight % |
|---|---|
| Solid dispersion | 35.29 |
| Methocel K15M | 30.00 |
| Methocel K100LV | 0.1 |
| Avicel PH101 | 33.35 |
| Magnesium stearate | 0.86 |
| Aerosil 200 | 0.4 |

The tablet blend was tabletted to a mean hardness of 73N on a Fette E1 tablet press. The *in vitro* dissolution for the tablets was determined under the conditions as specified in Example 1. The following results were obtained:

| Time(hours) | % Release |
|---|---|
| 1.0 | 9.8 |
| 2.0 | 19.3 |
| 4.0 | 41.7 |
| 6.0 | 55.8 |
| 8.0 | 67.5 |
| 10.0 | 77.3 |
| 12.0 | 88.6 |
| 24.0 | 103.9 |

### Example 4

### Preparation of solid dispersion

The solid dispersion corresponded to that prepared in Example 3.

### Preparation of tablet

A tablet formulation was prepared by blending the solid dispersion with the following tablet ingredients in the indicated proportions:

| Ingredient | Weight % |
|---|---|
| Solid dispersion | 35.29 |
| Methocel K15M | 0.10 |
| Methocel K100LV | 40.00 |
| Avicel PH101 | 23.35 |
| Magnesium stearate | 0.86 |
| Aerosil 200 | 0.40 |

The tablet blend was tabletted to a mean hardness of 73N on a Fette E1 tablet press. The *in vitro* dissolution for the tablets was determined under the conditions as specified in Example 1. The following results were obtained:

| Time(hours) | % Release |
|---|---|
| 1.0 | 22.2 |
| 2.0 | 38.5 |
| 4.0 | 67.9 |
| 6.0 | 92.1 |
| 8.0 | 103.1 |
| 10.0 | 105.6 |
| 12.0 | 105.5 |
| 24.0 | 106.2 |

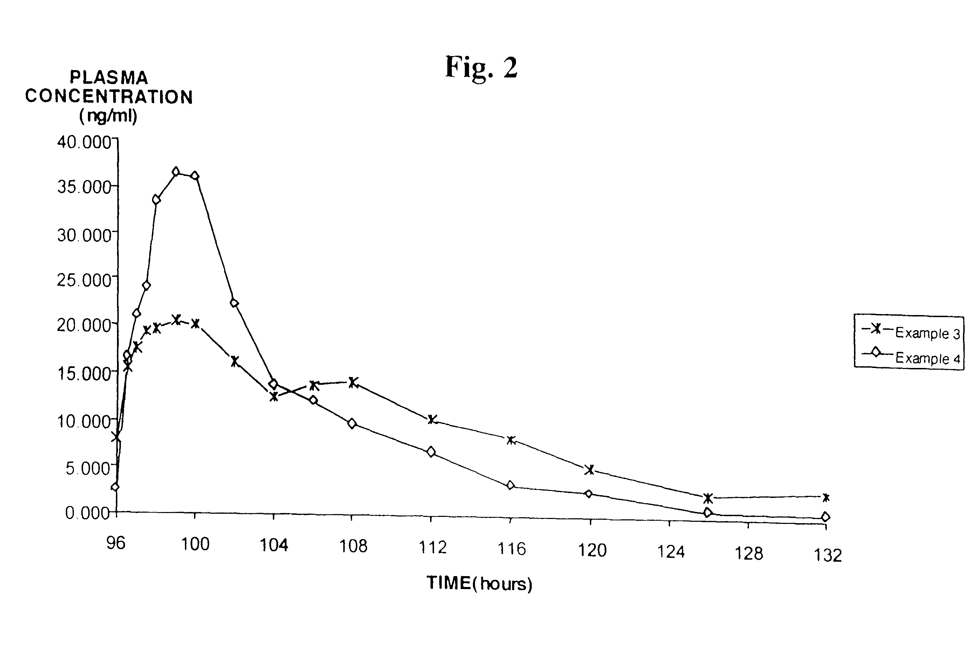

A comparison of the pharmacokinetic data for the formulations prepared in Examples 3 and 4 is shown in Table 2 and accompanying Fig. 2.

**Table 2**

| Parameter | Example 3 | Example 4 |
|---|---|---|
| AUCss | 305.22 ± 132.25 | 321.34 ± 109.66 |
| Cmax | 23.38 ± 8.75 | 43.04 ± 15.40 |
| Tmax | 3.70 ± 2.63 | 2.75 ± 0.98 |

Example 3 which uses a high percentage of a high viscosity Methocel (relative to Example 4) shows a slower dissolution profile and consequently shows a flatter *in vivo* plasma profile, thus demonstrating the changes in profile that can be achieved with different grades of Methocel.

### Example 5

### Preparation of solid dispersion

Lutrol F127 (4500g) was melted by heating to 80°C. Nifedipine (1500g) was gradually added to, and dispersed in, the molten Lutrol. Mixing was continued for 2 hrs before the solid dispersion was allowed to cool at room temperature, followed by milling.

### Preparation of tablet

A tablet formulation was prepared by blending a solid dispersion with the following tablet ingredients in the indicated proportions:

| Ingredient | Weight % |
|---|---|
| Solid dispersion | 35.29 |
| Methocel K15M | 15.00 |
| Methocel K100LV | 0.1 |
| Avicel PH101 | 47.95 |
| Magnesium stearate | 0.86 |
| Aerosil 200 | 0.8 |

The tablet blend was tabletted to a mean hardness of 90N on a Fette E1 tablet press. The *in vitro* dissolution for the tablets was determined under the conditions as specified in Example 1. The following results were obtained:

| Time(hours) | % Release |
|---|---|
| 1.0 | 15.7 |
| 2.0 | 40.0 |
| 4.0 | 63.9 |
| 6.0 | 81.7 |
| 8.0 | 103.8 |
| 10.0 | 109.9 |

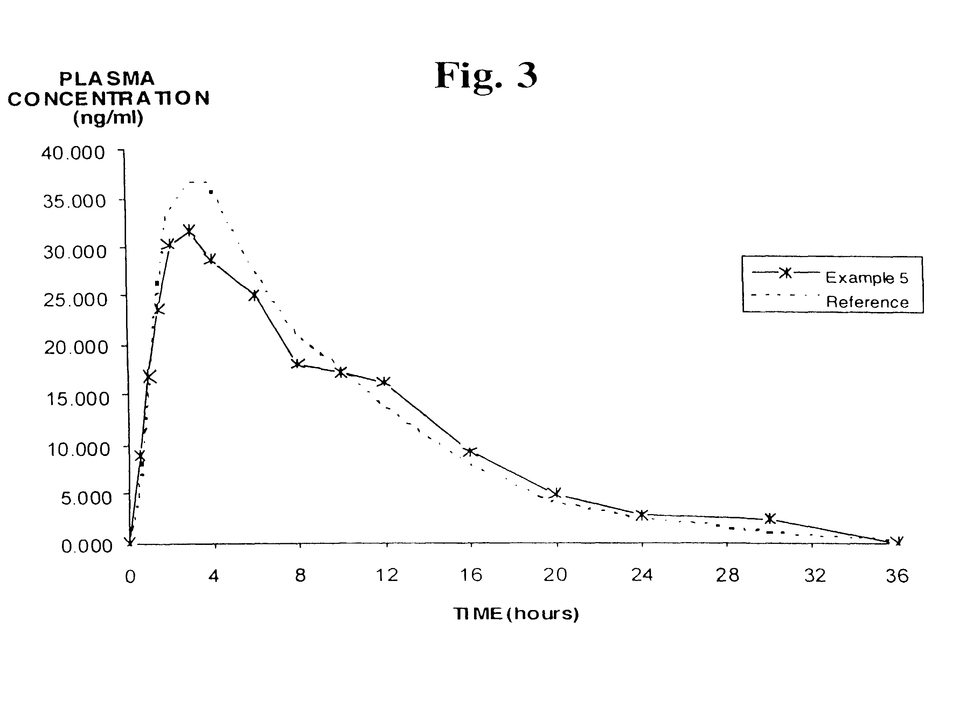

A comparison of the pharmacokinetic data for the formulation prepared in Example 5 with a commercially available once-daily nifedipine tablet (hereinafter referred to as reference) is shown in Table 3 and accompanying Fig. 3.

**Table 3**

| Parameter | Example 5 | Reference |
|---|---|---|
| AUC₍₀₋₃₆₎ | 374.93 ± 107.68 | 376.24 ± 135.45 |
| Cmax | 39.25 ± 13.54 | 40.87 ± 15.09 |
| Tmax | 3.60 ± 1.43 | 3.45 ± 1.17 |
| F % **** | 1.04 ± 0.22 | - |

| | | |
|---|---|---|
| **** F % = ratio of Example relative to reference. | | |

### Example 6

### Preparation of solid dispersion

Lutrol F127 (463 g) obtained from BASF (Ireland) Limited was melted by heating to 80°C. Cisapride (237 g) obtained from Janssen Pharmaceutica N.V., tartaric acid (150 g), obtained from R.B. Chemicals, and lactose (150 g) obtained from Forum Chemicals, were gradually added to, and dispersed in, the molten Lutrol. Mixing was continued for 0.5 hours until the last of the lactose had been added. The resulting solid dispersion was allowed to cool at room temperature, followed by milling thereof.

### Preparation of tablet

A tablet formulation was prepared by blending the solid dispersion with the following tabletting ingredients in the indicated proportions:

| Ingredient | Weight % |
|---|---|
| Solid dispersion | 24.2 |
| Methocel K15M | 20.0 |
| Avicel pH101 | 55.3 |
| Magnesium stearate | 0.5 |

The tablet blend was tabletted to a mean hardness of 129 N in a Killian RTS tablet press.

The *in vitro* dissolution for the tablets so prepared was determined using the standard U.S.P. apparatus II (paddles) operating at 50 r.p.m. using a 0.01 M HCl dissolution medium, volume 900 ml and temperature 37°C ± 0.5°C. At the indicated sampling times a 6.0 ml aliquot was removed and filtered through a 0.45 µm filter. A 4/10 dilution with 0.01 M HCl was performed and the absorbance at 270 nm measured against 0.01 M HCl. The following results were obtained:

| Time (hours) | % Release |
|---|---|
| 0.5 | 13.8 |
| 1.0 | 16.7 |
| 2.0 | 22.2 |
| 4.0 | 33.0 |
| 6.0 | 41.5 |
| 8.0 | 49.9 |
| 10.0 | 57.0 |
| 12.0 | 61.3 |
| 16.0 | 67.3 |
| 24.0 | 81.2 |

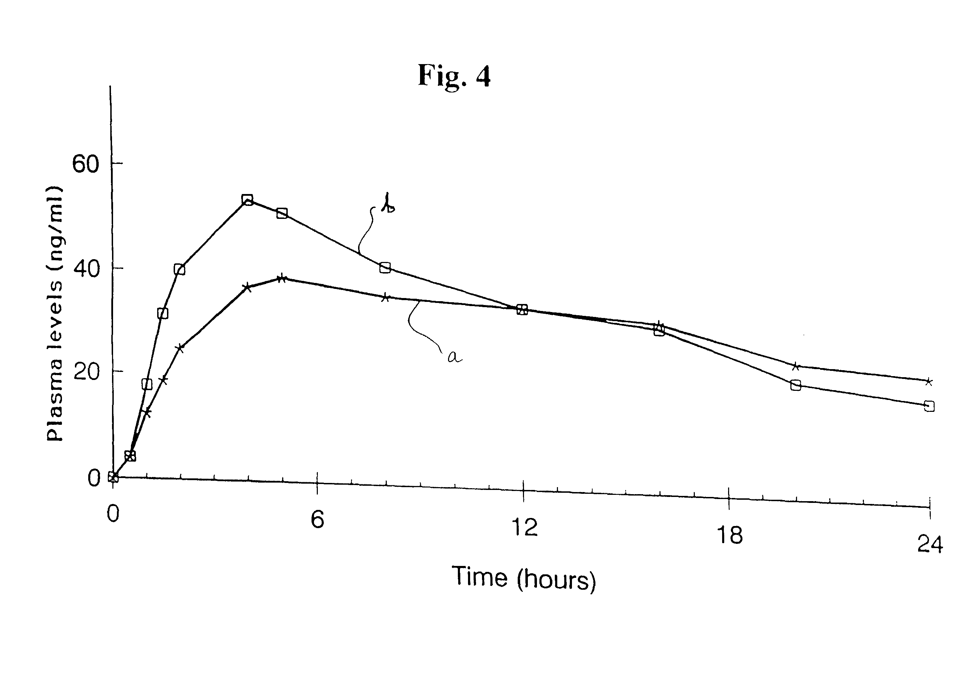

Plasma levels of cisapride achievable with the tablet formulation prepared above was assessed in 10 healthy male volunteer subjects. The results are shown in accompanying Fig. 4, wherein curve a) corresponds to the present Example.

### Example 7

### Preparation of solid dispersion

Lutrol F127 (463 g) was melted by heating to 80°C. Cisapride (237 g), tartaric acid (150 g), and lactose (150 g), were gradually added to, and dispersed in, the molten Lutrol. Mixing was continued for 0.5 hours until the last of the lactose had been added. The resulting solid dispersion was allowed to cool at room temperature, followed by milling thereof.

### Preparation of tablet

A tablet formulation was prepared by blending the solid dispersion with the following tabletting ingredients in the indicated proportions:

| Ingredient | Weight % |
|---|---|
| Solid dispersion | 24.2 |
| Methocel K100LV | 40.0 |
| Avicel pH101 | 35.3 |
| Magnesium stearate | 0.5 |

The tablet blend was tabletted to a mean hardness of 139 N in a Killian RTS tablet press.

The *in vitro* dissolution for the tablets so prepared was determined under the conditions specified in Example 6. The following results were obtained:

| Time (hours) | % Release |
|---|---|
| 0.5 | 8.4 |
| 1.0 | 12.0 |
| 2.0 | 21.3 |
| 4.0 | 43.4 |
| 6.0 | 54.1 |
| 8.0 | 70.5 |
| 10.0 | 87.6 |
| 12.0 | 98.7 |
| 16.0 | 108.3 |
| 24.0 | 112.5 |

Plasma levels of cisapride achievable with the tablet formulation prepared in accordance with the present Example was assessed in 10 healthy male volunteer subjects as before. The results are shown in Fig. 4, wherein curve b) corresponds to the present Example.

In the *in vivo* studies based on the tablet formulations of Examples 6 and 7 each of 10 subjects received one 40 mg tablet once a day.

It will be clear from accompanying Fig. 4 that the tablet formulations of Examples 6 and 7 have an extended absorption and plasma level profile. Each product appears to show an absorption phase with an initial more rapid absorption rate (over the first 4 hours) and a slower absorption rate over the remaining 12-16 hours.

The main difference between the plasma level profile of the product of Example 6 relative to the product of Example 7 is that the latter product has a more dominant peak concentration (C max) while the former product has the more extended absorption and plasma level profile. The respective products, while sharing a common solid dispersion formulation, do differ in release rate characteristics and in the viscosity grade of the cellulose-based polymer used to achieve rate control.

A summary of the pharmacokinetic parameters for the *in vivo* data for Examples 6 and 7 is shown in Table 4.

**Table 4**

| Parameter | Example 6 (40 mg) | Example 7 (40 mg) |
|---|---|---|
| AUC (0-infinity) ngxh/ml | 1148.10 ± 595.10 | 1125.85 ± 276.37 |
| Cmax ng/ml | 44.14 ± 16.01 | 55.71 ± 9.72 |
| Tmax hours | 7.30 ± 4.72 | 5.70 ± 3.83 |
| Kel*****1/hour | 0.082 ± 0.027 | 0.079 ± 0.023 |
| T1/2****** hours | 9.24 ± 2.78 | 9.78 ± 4.13 |

| | | |
|---|---|---|
| ***** The elimination rate constant | | |
| ****** The half-life of the drug in the plasma. | | |

### Example 8

### Preparation of solid dispersion

Lutrol F68 (2087.4g) was melted by heating to 80°C. Cisapride (522.4g) and tartaric acid (391.4g) were gradually added to, and dispersed in, the molten Lutrol. Mixing was continued for one hour before the solid dispersion was allowed to cool at room temperature, followed by milling thereof.

### Preparation of tablet

A tablet formulation was prepared by blending the solid dispersion with the following tabletting ingredients in the indicated proportions:

| Ingredient | Weight % |
|---|---|
| Solid dispersion | 32.8 |
| Methocel K100LV | 40.0 |
| Methocel K15M | 0.1 |
| Avicel PH101 | 26.1 |
| Aerosil 200 | 0.5 |
| Magnesium stearate | 0.5 |

The tablet blend was tabletted to a mean hardness of 125N in a Killian RTS tablet press. The *in vitro* dissolution for the tablets was determined under the conditions specified in Example 6. The following results were obtained:

| Time (hours) | % Release |
|---|---|
| 0.5 | 6.3 |
| 1.0 | 8.5 |
| 2.0 | 14.4 |
| 4.0 | 28.6 |
| 6.0 | 37.9 |
| 8.0 | 51.1 |
| 10.0 | 60.7 |
| 24.0 | 104.6 |

### Example 9

### Preparation of solid dispersion

Tartaric acid (156.4g) was dissolved in ethanol (833g). In a separate vessel the Cisapride (500g) was dissolved in acetone (5000g) followed by the Lutrol F68 (2001.7g). The two solutions were mixed at 50°C and solvent was evaporated under vacuum at elevated temperatures. The resulting molten solid dispersion was allowed to cool at room temperature, followed by milling thereof.

### Preparation of tablet

A tablet formulation was prepared by blending the solid dispersion with the following tabletting ingredients in the indicated proportions:

| Ingredient | Weight % |
|---|---|
| Solid dispersion | 30.35 |
| Methocel K100LV | 5.0 |
| Methocel K15M | 10.0 |
| Avicel PH101 | 53.65 |
| Aerosil 200 | 0.5 |
| Magnesium stearate | 0.5 |

The tablet blend was tabletted to a mean hardness of 108N in a Killian RTS tablet press. The *in vitro* dissolution for the tablets was determined under the conditions specified in Example 6. The following results were obtained:

| Time (hours) | % Release |
|---|---|
| 0.5 | 6.6 |
| 1.0 | 10.2 |
| 2.0 | 16.7 |
| 4.0 | 26.1 |
| 6.0 | 32.8 |
| 8.0 | 40.0 |
| 10.0 | 44.3 |
| 24.0 | 84.1 |

### Example 10

### Preparation of solid dispersion

The solid dispersion used was the solid dispersion of Example 9.

### Preparation of tablet

A tablet formulation was prepared by blending the solid dispersion with the following tabletting ingredients in the indicated proportions:

| Ingredient | Weight % |
|---|---|
| Solid dispersion | 30.35 |
| Methocel K100LV | 40.0 |
| Methocel K15M | 0.1 |
| Avicel PH101 | 28.55 |
| Aerosil 200 | 0.5 |
| Magnesium stearate | 0.5 |

The tablet blend was tabletted to a mean hardness of 115N in a Killian RTS tablet press. The *in vitro* dissolution for the tablets was determined under the conditions specified in Example 6. The following results were obtained:

| Time (hours) | % Release |
|---|---|
| 0.5 | 5.0 |
| 1.0 | 9.5 |
| 2.0 | 16.0 |
| 4.0 | 28.4 |
| 6.0 | 40.1 |
| 8.0 | 49.5 |
| 10.0 | 65.7 |
| 24.0 | 103.9 |

### Example 11 (Comparative Example)

### Preparation of solid dispersion

The solid dispersion used was the solid dispersion of Example 9.

### Preparation of mini-tablets

A mini-tablet formulation was prepared by blending the solid dispersion with the following tabletting ingredients in the indicated proportions:

| Ingredient | Weight % |
|---|---|
| Solid dispersion | 29.57 |
| Avicel PH101 | 58.93 |
| Aerosil 200 | 2.0 |
| Magnesium stearate | 0.5 |

The tablet blend was tabletted to a mean hardness of 75N in a Killian RTS tablet press.

### Coating of mini-tablets

A 500g quantity of the above mini-tablets was coated with the following coating solution in a Hi-Coater (trade mark) to a weight gain of 10.7%.

| Ingredient | Weight % |
|---|---|
| Eudragit L12.5 | 50.0 |
| Diethylphthalate | 1.25 |
| Talc | 1.50 |
| Isopropanol | 44.4 |
| Purified Water | 2.85 |

A further 500g of the mini-tablets were coated with the following coating solution in a Hi-Coater to a weight gain of 8.6%.

| Ingredient | Weight % |
|---|---|
| Eudragit S12.5 | 50.0 |
| Diethylphthalate | 1.25 |
| Talc | 1.55 |
| Isopropanol | 44.35 |
| Purified water | 2.85 |

### Final dosage form

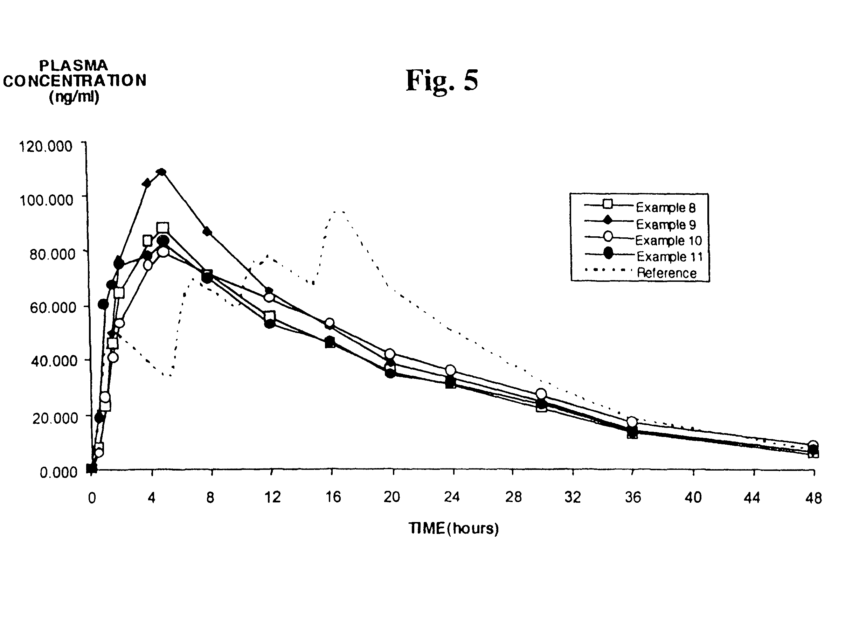

The final dosage form was a double zero capsule filled with three uncoated mini-tablets, three Eudragit L coated mini-tablets and three Eudragit S coated mini-tablets. This formulation provided a smooth plasma profile over an extended period as shown in Figure 5.

The uncoated mini-tablets showed rapid disintegration in acid (< 7 minutes) and 95% release on dissolution in 0.01N HCl. The enteric coated tablets showed no disintegration in acid (up to 60 minutes) and 100% release on dissolution in pH 6.8 buffer with SLS.

A summary of pharmacokinetic data for the formulations prepared in Examples 8-11 and the product sold under the trade mark Prepulsid (hereinafter referred to as reference) is presented in Table 5 and accompanying Fig. 5.

**Table 5**

| Parameter | Example 8 | Example 9 | Example 10 | Example 11 | Reference |
|---|---|---|---|---|---|
| AUC (Infinity) | 2027.58 ± 554.90 | 1747.99 ± 765.87 | 1982.09 ± 860.97 | 1820.12 ± 619.91 | 2124.80 ± 650.96 |
| Cmax | 111.54 ± 31.34 | 92.36 ± 40.51 | 84.24 ± 21.86 | 85.81 ± 22.27 | 101.94 ± 24.45 |
| Tmax | 4.30 ± 0.95 | 4.20 ± 1.23 | 7.40 ± 4.25 | 3.90 ± 1.60 | 13.55 ± 3.7 |
| F% | 99.2 ± 27.3 | 82.2 ± 23.1 | 94.7 ± 31.0 | 88.6 ± 28.5 | |

## Claims

1. A controlled release formulation for oral administration, comprising a solid dispersion of an active ingredient, which has poor acqueous solubility, in a hydrophilic poloxamer polymer, said solid dispersion being blended with a water swellable hydroxypropylmethylcellulose, a 2% solution of which has a viscosity in the range 100-100,000 centipoise, to form a hydrogel matrix and one or more tabletting ingredients to form a tablet core, said tablet core as such or following coating of the core with a polymeric coating being effective to achieve therapeutic levels of said active ingredient over extended periods of time following oral administration.

2. A formulation according to Claim 1, wherein the solid dispersion includes a surfactant component.

3. A formulation according to Claim 2, wherein the surfactant component is elected from sodium lauryl sulphate, a sodium carboxylate, an alkyl sulphate, a polyethylene glycol ester, a polyethylene ether, a sorbitan ester, an ethoxylated sorbitan ester and an alkyl trimethylammonium halide and a mixture thereof.

4. A formulation according to any preceding claim, wherein the solid dispersion includes an acid component.

5. A formulation according to Claim 4, wherein the acid component is selected from adipic acid, ascorbic acid, citric acid, fumaric acid, malic acid, succinic acid and tartaric acid.

6. A formulation according to any one of Claims 1-3, wherein the solid dispersion includes a base component.

7. A formulation according to Claim 6, wherein the base component is selected from calcium carbonate, calcium hydroxide, magnesium hydroxide, sodium bicarbonate, sodium carbonate, sodium citrate and sodium hydroxide.

8. A formulation according to any one of Claims 2-7, wherein the surfactant, acid or base component is present in a ratio of 0.01:1.0 to 5.0:1.0 by weight of the active ingredient.

9. A formulation according to any preceding claim, wherein the active ingredient and the poloxamer are present in an amount of 0.1:1.0 to 10.0:1.0 by weight.

10. A formulation according to any preceding claim, wherein the poloxamer contains between 60% and 90% by weight of polyoxyethylene.

11. A formulation according to any preceding claim, which is in a multi-particulate form.

12. A formulation according to Claim 11, which is in the form of mini-tablets.

13. A formulation according to Claim 11 or 12, which includes an amount of said solid dispersion for immediate release.

14. A formulation according to any preceding claim, wherein the hydroxypropylmethylcellulose is present in an amount of 5-75% by weight.

15. A formulation according to any preceding claim, wherein the core is surrounded by a multi-porous, rate-controlling membrane.

16. A formulation according to any preceding claim, wherein the active ingredient is selected from cisapride, cyclosporin, diclofenac, felodipine, ibuprofen, indomethacin, nicardipine, nifedipine, terfenadine and theophylline.

## Patentansprüche

1. Formulierung zur oralen Verabreichung mit kontrollierter Wirkstofffreisetzung, enthaltend eine feste Dispersion eines schlecht wasserlöslichen Wirkstoffs in einem hydrophilen Poloxamer-Polymeren, wobei die feste Dispersion mit einer wasserquellfähigen Hydroxypropylmethylcellulose vermischt ist, wovon eine 2-%ige Lösung eine Viskosität im Bereich von 100-100000 Centipoise aufweist, zur Bildung einer Hydrogelmatrix und einen oder mehrere Tablettierbestandteile zur Bildung eines Tablettenkerns, wobei der Tablettenkern als solcher oder nach dem Überziehen des Kerns mit einem Polymerüberzug zum Erzielen therapeutischer Konzentrationen des Wirkstoffs über längere Zeiträume nach oraler Verabreichung wirksam ist.

2. Formulierung nach Anspruch 1, wobei die feste Dispersion eine oberflächenaktive Komponente enthält.

3. Formulierung nach Anspruch 2, wobei die oberflächenaktive Komponente ausgewählt ist aus Natriumlaurylsulfat, einem Natriumcarboxylat, einem Alkylsulfat, einem Polyethylenglycolester, einem Polyethylenether, einem Sorbitanester, einem ethoxylierten Sorbitanester und einem Alkyltrimethylammoniumhalogenid und einem Gemisch hiervon.

4. Formulierung nach einem vorhergehenden Anspruch, wobei die feste Dispersion eine Säure-Komponente enthält.

5. Formulierung nach Anspruch 4, wobei die Säure-Komponente aus Adipinsäure, Ascorbinsäure, Citronensäure, Fumarsäure, Äpfelsäure, Bernsteinsäure und Weinsäure ausgewählt ist.

6. Formulierung nach einem der Ansprüche 1-3, wobei die feste Dispersion eine Basen-Komponente enthält.

7. Formulierung nach Anspruch 6, wobei Basen-Komponente aus Calciumcarbonat, Calciumhydroxid, Magnesiumhydroxid, Natriumbicarbonat, Natriumcarbonat, Natriumcitrat und Natriumhydroxid ausgewählt ist.

8. Formulierung nach einem der Ansprüche 2-7, wobei das oberflächenaktive Mittel, die Säure- oder die Basen-Komponente in einem Verhältnis von 0,01:1,0 bis 5,0:1,0 bezogen auf das Gewicht des Wirkstoffs vorhanden sind.

9. Formulierung nach einem vorhergehenden Anspruch, wobei der Wirkstoff und das Poloxamere in einer Menge von 0,1:1,0 bis 10,0:1,0 bezogen auf das Gewicht vorhanden sind.

10. Formulierung nach einem vorhergehenden Anspruch, wobei das Poloxamere zwischen 60 und 90 Gew.-% Polyoxyethylen enthält.

11. Formulierung nach einem vorhergehenden Anspruch, die in einer Mehrteilchenform vorliegt.

12. Formulierung nach Anspruch 11, die in Form von Minitabletten vorliegt.

13. Formulierung nach Anspruch 11 oder 12, die eine Menge der festen Dispersion zur sofortigen Freisetzung enthält.

14. Formulierung nach einem vorhergehenden Anspruch, wobei die Hydroxypropylmethylcellulose in einer Menge von 5-75 Gew.-% vorhanden ist.

15. Formulierung nach einem vorhergehenden Anspruch, wobei der Kern von einer multiporösen, geschwindigkeitskontrollierenden Membran umgeben ist.

16. Formulierung nach einem vorhergehenden Anspruch, wobei der Wirkstoff aus Cisaprid, Cyclosporin, Diclofenac, Felodipin, Ibuprofen, Indomethacin, Nicardipin, Nifedipin, Terfenadin und Theophyllin ausgewählt ist.

## Revendications

1. Formulation retard destinée à l'administration par voie orale, qui comprend une dispersion solide, dans un polymère poloxamer hydrophile, d'un principe actif présentant une faible solubilité dans l'eau, ladite dispersion solide étant mélangée avec de l'hydroxypropylméthylcellulose capable de gonfler dans l'eau, dont une solution à 2 % présente une viscosité comprise dans la gamme de 100-100 000 centipoises, pour former une matrice d'hydrogel, et un ou plusieurs ingrédients de pastillage permettant de former le noyau d'un comprimé, ledit noyau de comprimé permettant de manière efficace, tel quel ou après enrobage du noyau avec un revêtement polymère, d'atteindre des taux thérapeutiques dudit principe actif pendant des durées prolongées après administration par voie orale.

2. Formulation suivant la revendication 1, dans laquelle la dispersion solide comprend un composant tensioactif.

3. Formulation selon la revendication 2, dans laquelle le composant tensioactif est choisi parmi le laurylsulfate de sodium, un carboxylate de sodium, un sulfate d'alkyle, un ester de polyéthylèneglycol, un polyéthylène éther, un ester de sorbitan, un ester de sorbitan éthoxylé et un halogénure d'alkyltriméthylammonium, et un mélange de ces composés.

4. Formulation selon l'une quelconque des revendications précédentes, dans laquelle la dispersion solide comprend un composant acide.

5. Formulation selon la revendication 4, dans laquelle le composant acide est choisi parmi l'acide adipique, l'acide ascorbique, l'acide citrique, l'acide fumarique, l'acide malique, l'acide succinique et l'acide tartrique.

6. Formulation selon l'une quelconque des revendications 1 à 3, dans laquelle la dispersion solide comprend un composant basique.

7. Formulation selon la revendication 6, dans laquelle le composant basique est choisi parmi le carbonate de calcium, l'hydroxyde de calcium, l'hydroxyde de magnésium, le bicarbonate de sodium, le carbonate de sodium, le citrate de sodium et l'hydroxyde de sodium.

8. Formulation selon l'une quelconque des revendications 2 à 7, dans laquelle le tensioactif, le composant acide ou le composant basique est présent dans un rapport de 0,01:1,0 à 5,0:1,0 en poids par rapport au principe actif.

9. Formulation selon l'une quelconque des revendications précédentes, dans laquelle le principe actif et le poloxamer sont présents dans un rapport de 0,1:1,0 à 10,0:1,0 en poids.

10. Formulation selon l'une quelconque des revendications précédentes, dans laquelle le poloxamer contient de 60 % à 90 % en poids de polyoxyéthylène.

11. Formulation selon l'une quelconque des revendications précédentes, ayant une forme multiparticulaire.

12. Formulation selon la revendication 11, qui est sous la forme de mini-comprimés.

13. Formulation selon la revendication 11 ou 12, qui comprend une certaine quantité de ladite dispersion solide pour une libération immédiate.

14. Formulation selon l'une quelconque des revendications précédentes, dans laquelle l'hydroxypropylméthylcellulose est présente en une proportion de 5-75 % en poids.

15. Formulation selon l'une quelconque des revendications précédentes, dans laquelle le noyau est entouré d'une membrane poreuse, contrôlant la vitesse de libération.

16. Formulation selon l'une quelconque des revendications précédentes, dans laquelle le principe actif est choisi parmi le cisapride, la cyclosporine, le diclofénac, la félodipine, l'ibuprofène, l'indométhacine, la nicardipine, la nifédipine, la terfénadine et la théophylline.
